(19) Europäisches Patentamt / European Patent Office / Office européen des brevets

(11) **EP 4 435 676 A1**

(12) **EUROPEAN PATENT APPLICATION**

(43) Date of publication:
**25.09.2024 Bulletin 2024/39**

(21) Application number: **24161994.9**

(22) Date of filing: **07.03.2024**

(51) International Patent Classification (IPC):
**G06N 10/20** *(2022.01)* **G06N 10/60** *(2022.01)*

(52) Cooperative Patent Classification (CPC):
**G06N 10/20; G06N 10/60**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.03.2023 JP 2023047321**

(71) Applicants:
• **FUJITSU LIMITED**
  **Kawasaki-shi, Kanagawa 211-8588 (JP)**

• **Qunasys Inc.**
  **Tokyo 113-0001 (JP)**

(72) Inventors:
• **Kanasugi, Shota**
  **Kawasaki-shi, Kanagawa, 211-8588 (JP)**
• **Nakagawa, Yuya**
  **Bunkyo-ku, Tokyo, 113-0001 (JP)**
• **Tsutsui, Shoichiro**
  **Bunkyo-ku, Tokyo, 113-0001 (JP)**

(74) Representative: **Hoffmann Eitle**
**Patent- und Rechtsanwälte PartmbB**
**Arabellastraße 30**
**81925 München (DE)**

(54) **COMPUTER-READABLE RECORDING MEDIUM, INFORMATION PROCESSING METHOD, AND INFORMATION PROCESSING DEVICE**

(57)   A computer-readable recording medium stores a program for causing a computer to execute a process including: when calculating a Green's function by using expected values represented respectively by quantum circuits corresponding to a specific term forming the Green's function expressed in qubits and used when analyzing characteristics of a substance, selecting a first quantum circuit from a group into which two or more of the quantum circuits are classified when the quantum circuits are classified such that quantum circuits thereof corresponding to a same expected value are classified into a same group according to at least one of multiple symmetry characteristics of the substance; and controlling a computing device to calculate the Green's function so that, by measuring a first expected value represented by the first quantum circuit, the computing device omits measuring a second expected value represented by a second quantum circuit different from the first quantum circuit in the group.

FIG.1

**Description**

FIELD OF THE INVENTION

Embodiments discussed herein relate to a computer-readable recording medium, an information processing method, and an information processing device.

BACKGROUND OF THE INVENTION

Up until now, in the fields of materials development, drug discovery research, etc., simulations of quantum many-body systems related to substances such as polymers or solid crystals have occasionally been carried out to analyze the characteristics of the substances. For example, in some cases, quantum many-body system simulation related to a solid material has been carried out by calculating a Green's function of a solid material.

According to one prior art, for example, based on the expected value of the spin operator calculated by a quantum computer, a classical computer calculates elements of the Hamiltonian matrix equivalent to the Hamiltonian operator and elements of the overlap matrix, to calculate a one-electron Green's function. Another prior art is, for example, a technique of identifying a linear sum of unitary operators based on calculation results of quantum gates that execute predetermined calculations based on parameters included in n-layer parameters that are set based on coefficients of the linear sum of the unitary operators. For example, refer to Japanese Laid-Open Patent Publication No. 2021-068281 and Japanese Laid-Open Patent Publication No. 2022-007590.

SUMMARY OF THE INVENTION

It is an object in one aspect of the embodiments to at least solve the above problems in the conventional technologies.

According to an aspect of an embodiment, a computer-readable recording medium stores therein an information processing program for causing a computer to execute a process, the process including: when calculating a Green's function by using a plurality of expected values represented respectively by a plurality of quantum circuits corresponding to a specific term that forms the Green's function, which is expressed in qubits and used when analyzing characteristics of a substance, selecting a first quantum circuit from a group into which two or more of the plurality of quantum circuits are classified when the plurality of quantum circuits is classified such that among the plurality of quantum circuits, quantum circuits corresponding to a same expected value are classified in a same group according to at least one of a plurality of characteristics of the substance, the plurality of characteristics including space-group symmetry, gauge symmetry, particle-hole symmetry, and time-reversal symmetry; and controlling a computing device to calculate the Green's function so that, by measuring a first expected value represented by the selected first quantum circuit, the computing device omits measuring a second expected value represented by a second quantum circuit different from the selected first quantum circuit in the group.

BRIEF DESCRIPTION OF DRAWINGS

Fig. 1 is an explanatory view of a practical example of an information processing method according to an embodiment.
Fig. 2 is an explanatory view of an example of an information processing system 200.
Fig. 3 is a block diagram depicting an example of a hardware configuration of the information processing device 100.
Fig. 4 is a block diagram depicting an example of a hardware configuration of a quantum computer 201.
Fig. 5 is a block diagram of an example of a functional configuration of the information processing device 100.
Fig. 6 is an explanatory view of an example of a quantum circuit 600.
Fig. 7 is an explanatory view of an example of classifying two or more quantum circuits 600 corresponding to a same expected value into a same group.
Fig. 8 is an explanatory view of an example of selecting a quantum circuit 600 from a group.
Fig. 9 is an explanatory view of an example of an advantageous effect.
Fig. 10 is an explanatory view of an example of an advantageous effect.
Fig. 11 is an explanatory view of an example of an advantageous effect.
Fig. 12 is a flowchart of an example of an overall processing procedure.

DESCRIPTION OF THE INVENTION

First, problems associated with the conventional techniques are discussed. In the prior arts, however, it is difficult to carry out the quantum many-body system simulations. Although it is conceivable, for example, to calculate the Green's function of a solid material by using a classical computer, the amount of resources required when calculating the Green's function of the solid material tends to increase exponentially. On the other hand, it is conceivable, for example, to calculate the Green's function of a solid material by using a quantum computer, but the expected values of the multiple quantum circuits corresponding to a specific term that forms the Green's function have to be measured and thus, the number of measurements tends to become enormous.

Preferred embodiments of a computer-readable recording medium, an information processing method, and an information processing device according to the present invention are explained with reference to the accompanying drawings.

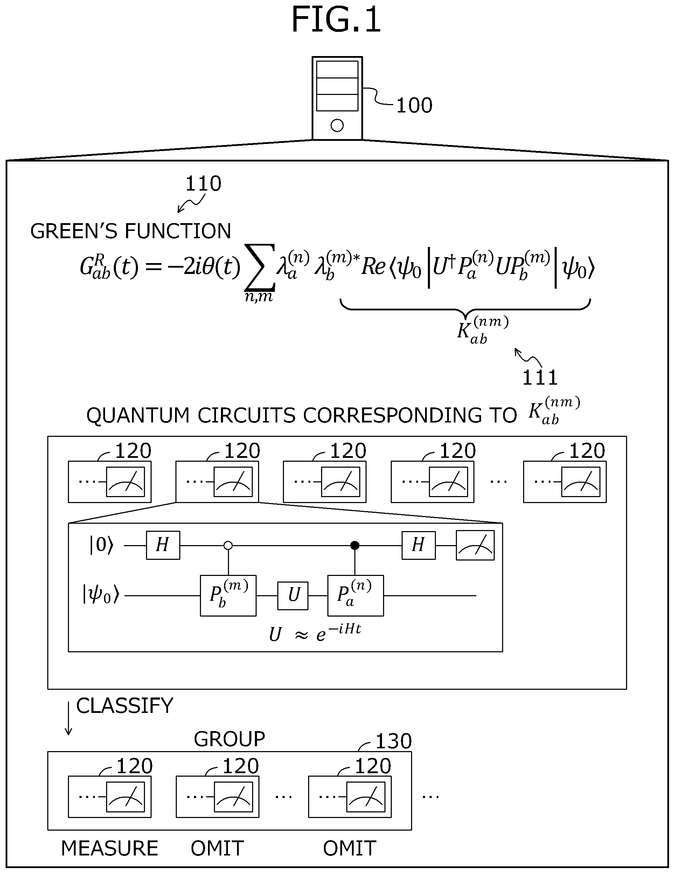

Fig. 1 is an explanatory view of a practical example of the information processing method according to the embodiment. An information processing device 100 is a computer that controls a computing unit that calculates a Green's function. The information processing device 100 is, for example, a server or a personal computer (PC).

The computing unit is, for example, another computer different from the information processing device 100. The computing unit is, for example, a quantum computer. The computing unit may be, for example, the information processing device 100.

A Green's function is used, for example, in the field of material development or drug discovery research. A Green's function is used, for example, mainly when analyzing characteristics of solid materials. It is conceivable, for example, to analyze characteristics of solid materials, based on a spectral function obtained by Fourie transforming the Green's function. The characteristics include, for example, quasiparticle spectrum, band structure, electron density, and response to electromagnetic fields.

A Green's function is expressed by, for example, formula (1) below, where H: Hamiltonian, t: time parameter, a and b: electron index, $c_a$: electron annihilation operator, $c_b\dagger$: electron generation operator, $\theta$: Heaviside step function, and $|\psi_0\rangle$: ground state of Hamiltonian H. In the following description, a Green's function at zero temperature is described for the sake of simplicity, but the same description is applicable also to a Green's function at finite temperature.

$$G_{ab}^{R}(t) = -i\theta(t)\langle\psi_0|\{e^{iHt}c_a e^{-iHt}, c_b^{\dagger}\}|\psi_0\rangle \tag{1}$$

The spectral function is expressed by, for example, formula (2) below, where k denotes a wavenumber.

$$A_k(\omega) = -\pi^{-1} Im G_k^R(\omega) \tag{2}$$

However, there is a problem in that a Green's function is difficult to calculate. For example, a case is conceivable where the Green's function is calculated by using a classical computer. In such a case, the amount of resources required for calculation tends to increase exponentially as the quantum system size increases. The amount of resources is, for example, a memory usage amount. It is therefore difficult to calculate a Green's function using a classical computer.

On the other hand, a case is conceivable, for example, where a Green's function is calculated by using a quantum computer. For example, a case is conceivable where the Jordan-Wigner transform of formula (3) below is used to express a Green's function through formula (4) below obtained by the qubit expression of the formula (1), to thereby calculate a Green's function using the quantum computer. Here, $\lambda_a^{(n)}$ is a complex constant. $P_a^{(n)}$ is a Pauli operator. U is a unitary gate that approximates a time evolution operator $e^{-iHt}$.

$$c_a \mapsto \sum_n \lambda_a^{(n)} P_a^{(n)} \tag{3}$$

$$G_{ab}^{R}(t) = -2i\theta(t)\sum_{n,m}\lambda_a^{(n)}\lambda_b^{(m)*}Re\langle\psi_0|U^{\dagger}P_a^{(n)}U P_b^{(m)}|\psi_0\rangle \tag{4}$$

In this case also, a Green's function is difficult to calculate. For example, when calculating a Green's function, it is necessary to measure expected values of multiple quantum circuits corresponding to a specific term expressed by $K_{ab}^{(nm)}$ of formula (5) below in the above formula (4). Thus, there is a tendency that as the quantum system size increases, the number of the quantum circuits increases and thus, the number of measurements becomes enormous. As a result, it difficult to calculate a Green's function by using a quantum computer.

$$K_{a,b}^{(n,m)}(t) = Re\langle\psi_0|U^\dagger P_a^{(n)} U P_b^{(m)}|\psi_0\rangle \qquad\qquad (5)$$

Thus, in the present embodiment, an information processing method is described that is capable of facilitating the calculation of a Green's function using a quantum computer.

In Fig. 1, the information processing device 100 stores a Green's function 110 expressed by qubits and used when analyzing characteristics of a substance. The Green's function 110 includes a specific term 111. The specific term 111 is, for example, $K_{ab}^{(nm)}$ of the formula (5) described above.

The information processing device 100 is assumed to be capable of controlling a computing unit 101 that calculates the Green's function 110. The computing unit 101 calculates the Green's function 110 by using an expected value represented respectively by quantum circuits 120 corresponding to the specific term 111. The computing unit 101 is a quantum computer different from the information processing device 100. The computing unit 101 may be included in the information processing device 100. The computing unit 101 may be an emulator of the quantum computer.

(1-1) Depending on the characteristics of a substance, the information processing device 100 stores any one of groups 130 in which two or more quantum circuits 120 are classified. Each of the groups 130 is an aggregate of two or more quantum circuits 120 classified when the quantum circuits 120 are classified such that the quantum circuits 120 corresponding to a same expected value among the quantum circuits 120 are classified into a same group 130.

The quantum circuits 120 corresponding to a same expected value are, for example, the quantum circuits 120 representing respective expected values whose absolute values are the same. The characteristics of a substance include, for example, at least any one of characteristics consisting of space-group symmetry, gauge symmetry, particle-hole symmetry, and time-reversal symmetry.

The information processing device 100 stores, for example, any one of the groups 130 set in advance. For example, the information processing device 100 may classify the quantum circuits 120 depending on the characteristics of a substance, to thereby identify and store the groups 130 in which two or more quantum circuits 120 are classified.

This enables the information processing device 100 to identify two or more quantum circuits 120 corresponding to a same expected value.

(1-2) The information processing device 100 selects a quantum circuit 120 from a stored group 130. The information processing device 100 controls the computing unit 101 so as to measure the expected value represented by the selected quantum circuit 120 and to omit processing of measuring the expected value represented by another quantum circuit 120 of the stored group 130. Another quantum circuit 120 is a quantum circuit 120 different from the selected quantum circuit 120.

The information processing device 100 controls the computing unit 101, for example, so that the measured expected value represented by the quantum circuit 120 is used also as expected values represented by the quantum circuits 120 different from the quantum circuit 120 and in the stored group 130.

As a result, with respect to a stored group 130, when the expected value represented by any quantum circuit 120 of the stored group 130 is measured, the information processing device 100 needs not measure an expected value represented by another quantum circuit 120 of the stored group 130 and different from the quantum circuit 120.

Thus, the information processing device 100 may reduce the number of measurements of the expected value in calculating the Green's function 110, to achieve a reduction in processing burden and processing time on the computing unit 101. The information processing device 100 allows the computing unit 101 to facilitate calculation of the Green's function 110.

However, since the information processing device 100 works based on characteristics of strict symmetry the Green's function 110 meets, the calculation result generally differs between a case of using the information processing device 100 and a case of not using the information processing device 100, if approximation breaking the original Hamiltonian symmetry is introduced when preparing the ground state $|\psi_0\rangle$ or the unitary gate U that approximates the time evolution operator.

Here, for simplification of description, how the information processing device 100 controls the computing unit 101 has been described by focusing on one group 130. It is preferred that when, for example, there are multiple groups 130 in which two or more quantum circuits 120 are classified, the information processing device 100 controls the computing unit 101 in such a manner as described above, for each of the groups 130. It is also conceivable that, for example, for a quantum circuit 120 which is not under control of the information processing device 100, the computing unit 101 independently measures the expected value represented by the quantum circuit 120.

Although here, a case has been described where the information processing device 100 controls the computing unit 101 solely, this is not limitative. For example, the information processing device 100 may cooperate with another computer to control the computing unit 101. For example, another computer may have a function of classifying the quantum circuits 120 depending on the characteristics of a substance and thus, the information processing device 100 receives from this other computer, a group 130 in which two or more quantum circuits 120 are classified. For example, multiple computers may implement the function of the information processing device 100 in cooperation. For example, the function of the information processing device 100 may be implemented on a cloud.

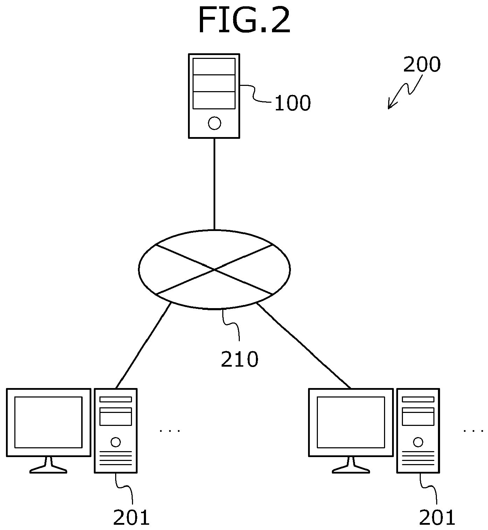

An example of an information processing system 200 to which the information processing device 100 of Fig. 1 is applied is described with reference to Fig. 2.

Fig. 2 is an explanatory view of an example of the information processing system 200. In Fig. 2, the information processing system 200 includes the information processing device 100 and a quantum computer 201.

In the information processing system 200, the information processing device 100 and the quantum computer 201 are connected via cable or a wireless network 210. The network 210 is, for example, a local area network (LAN), a wide area network (WAN), or the Internet.

The information processing device 100 is a computer that controls the quantum computer 201. The information processing device 100 stores a Green's function expressed by qubits. The Green's function includes a specific term. The specific term is, for example, $K_{ab}^{(nm)}$ of the formula (5) described above.

The information processing device 100 stores multiple groups into which quantum circuits are classified such that depending on the characteristics of a substance, quantum circuits corresponding to a same expected value among the quantum circuits corresponding to the specific term are classified into a same group. Included among the groups is at least one or more groups into which two or more quantum circuits are classified.

The characteristics of a substance include, for example, at least any one of characteristics consisting of space-group symmetry, gauge symmetry, particle-hole symmetry, and time-reversal symmetry.

The information processing device 100 stores, for example, preset groups. For example, depending on the characteristics of a substance, the information processing device 100 may classify the quantum circuits and thereby identify and store multiple groups.

For each of the groups, the information processing device 100 selects a quantum circuit from the group. The information processing device 100 indicates, in an identifiable manner, the identified groups and quantum circuits selected respectively from the identified groups, and generates a calculation request requesting calculation of the Green's function. The calculation request may contain the Green's function.

The information processing device 100 transmits the generated calculation request to the quantum computer 201. The information processing device 100 receives a calculation result of the Green's function from the quantum computer 201. The information processing device 100 outputs the calculation result of the Green's function in such a manner as to enable reference by the user. The information processing device 100 is, for example, a server or a PC.

The quantum computer 201 is a computer that calculates the Green's function. The quantum computer 201 receives a calculation request from the information processing device 100. Based on the calculation request, the quantum computer 201 identifies each of the groups and the quantum circuits selected respectively from the identified groups.

For each of the identified groups, the quantum computer 201 measures an expected value represented by a quantum circuit selected from the group. For each of the groups in which two or more quantum circuits are classified, the quantum computer 201 uses the measured expected value as expected values represented by quantum circuits in the group and different from the selected quantum circuit.

Thus, without measuring expected values represented by the quantum circuits different from the selected quantum circuit in a same group in which two or more quantum circuits are classified, the quantum computer 201 may make expected values represented respectively by all of the quantum circuits belonging to the group available. This enables the quantum computer 201 to reduce the number of measurements of expected values when calculating the Green's function, thereby facilitating the calculation of the Green's function.

The quantum computer 201 calculates the Green's function, based on an expected value represented by each of the quantum circuits corresponding to a specific term. The quantum computer 201 transmits the calculation result of the Green's function to the information processing device 100. Thus, the quantum computer 201 may calculate the Green's function. The quantum computer 201 may reduce the processing burden and the processing time in calculating the Green's function. The quantum computer 201 may make the calculation result of the Green's function available by the information processing device 100.

Although here, a case has been described where the information processing device 100 and the quantum computer 201 are different devices, this is not limitative. For example, the information processing device 100 may have the function of the quantum computer 201 and act as the quantum computer 201.

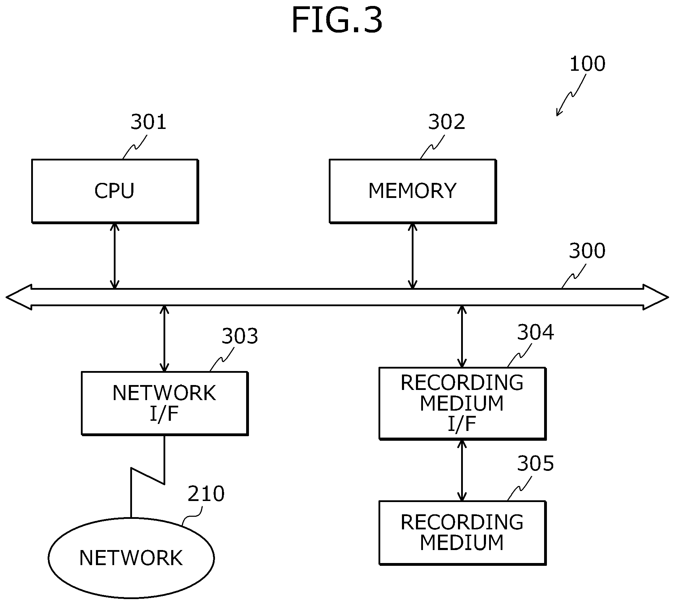

Next, an example of hardware configuration of the information processing device 100 is described with reference to Fig. 3.

Fig. 3 is a block diagram depicting an example of a hardware configuration of the information processing device 100. In Fig. 3, the information processing device 100 has a central processing unit (CPU) 301, a memory 302, a network interface (I/F) 303, a recording medium I/F 304, and a recording medium 305. Further, the components are connected by a bus 300.

Here, the CPU 301 governs overall control of the information processing device 100. The memory 302, for example, includes read-only memory (ROM), random access memory (RAM), and flash ROM, etc. In particular, for example, the flash ROM and/or ROM store various types of programs, and the RAM is used as a work area by the CPU 301. Programs stored to the memory 302 are loaded onto the CPU 301, whereby encoded processes are executed by the CPU 301.

The network I/F 303 is connected to a network 210 through a communications line and is connected to other computers via the network 210. Further, the network I/F 303 administers an internal interface with the network 210 and controls the input and output of data from other computers. The network I/F 303, for example, is a modem, a LAN adapter, etc.

The recording medium I/F 304, under the control of the CPU 301, controls the reading and writing of data with respect to the recording medium 305. The recording medium I/F 304 is, for example, a disk drive, a solid-state drive (SSD), a universal serial bus (USB) port, etc. The recording medium 305 is a non-volatile memory storing therein data written thereto under the control of the recording medium I/F 304. The recording medium 305 is, for example, a disk, a semiconductor memory, a USB memory, etc. The recording medium 305 may be removable from the information processing device 100.

The information processing device 100, in addition to the components described above, may have, for example, a keyboard, a mouse, a display, a printer, a scanner, a microphone, a speaker, etc. Further, the information processing device 100 may have the recording medium I/F 304 and/or the recording medium 305 in plural. Further, the information processing device 100 may omit the recording medium I/F 304 and/or the recording medium 305.

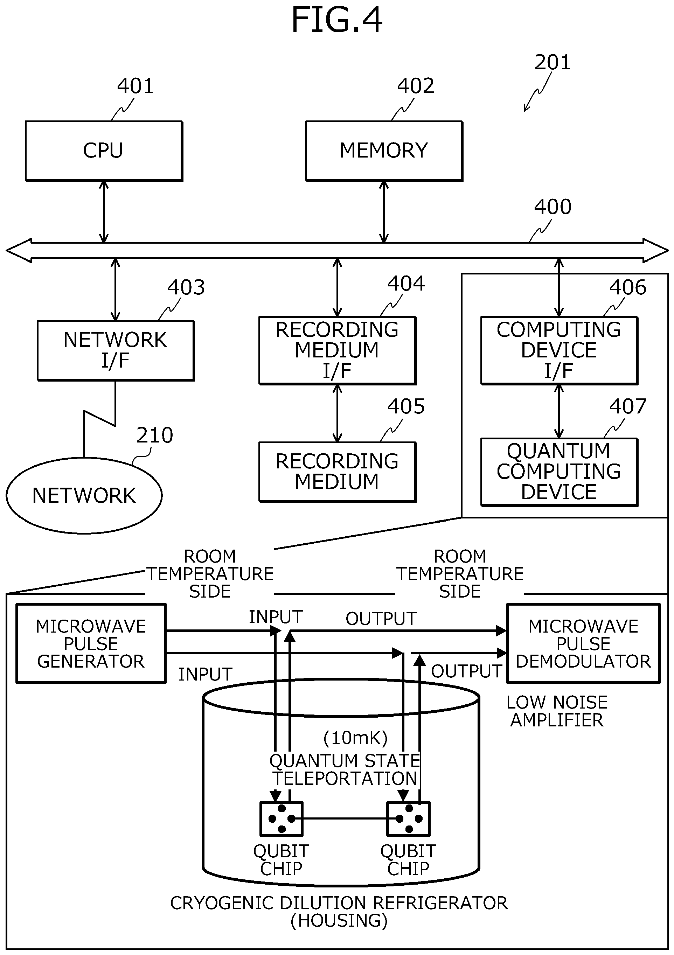

Next, an example of hardware configuration of the quantum computer 201 is described with reference to Fig. 4.

Fig. 4 is a block diagram depicting an example of a hardware configuration of the quantum computer 201. In Fig. 4, the quantum computer 201 has a central processing unit (CPU) 401, a memory 402, a network interface (I/F) 403, a recording medium I/F 404, and a recording medium 405. The quantum computer 201 may further have a computing device I/F 406 and a quantum computing device 407. Further, the components are connected by a bus 400.

Here, the CPU 401 governs overall control of the quantum computer 201. The memory 402, for example, includes ROM, RAM, and flash ROM, etc. In particular, for example, the flash ROM and/or ROM store various types of programs, and the RAM is used as a work area by the CPU 401. Programs stored to the memory 402 are loaded onto the CPU 401, whereby encoded processes are executed by the CPU 401.

The network I/F 403 is connected to a network 210 through a communications line and is connected to other computers via the network 210. Further, the network I/F 403 administers an internal interface with the network 210 and controls the input and output of data from other computers. The network I/F 403, for example, is a modem, a LAN adapter, etc.

The recording medium I/F 404, under the control of the CPU 401, controls the reading and writing of data with respect to the recording medium 405. The recording medium I/F 404 is, for example, a disk drive, a SSD, a USB port, etc. The recording medium 405 is a non-volatile memory storing therein data written thereto under the control of the recording medium I/F 404. The recording medium 405 is, for example, a disk, a semiconductor memory, a USB memory, etc. The recording medium 405 may be removable from the quantum computer 201.

The computing device I/F 406, under the control of the CPU 401, controls access to the quantum computing device 407. The computing device I/F 406 uses a microwave pulse generator to convert signals output from the CPU 401 into signals to be input to the quantum computing device 407 and transmits the signal to the quantum computing device 407. The computing device I/F 406 uses a microwave pulse demodulator to convert signals output from the quantum computing device 407 into signals to be input to the CPU 401 and transmits the signals to the CPU 401. The quantum computing device 407 is a computing device equipped with one or more qubit chips cooled to a cryogenic temperature of 10 mK. A qubit chip represents, for example, a logical qubit. The quantum computing device 407 utilizes one or more qubit chips to perform a predetermined computation according to a signal input thereto and outputs a signal corresponding to the result of the predetermined operation performed.

The quantum computer 201, in addition to the components described above, may have, for example, a keyboard, a mouse, a display, a printer, a scanner, a microphone, a speaker, etc. Further, the information processing device 100 may have the recording medium I/F 404 and/or the recording medium 405 in plural. Further, the quantum computer 201 may omit the recording medium I/F 404 and/or the recording medium 405. Further, the qubit chip in the quantum computing device 407 may be controlled by a method other than microwave. The qubit chip in the quantum computing device 407 may realize, for example, an optical qubit.

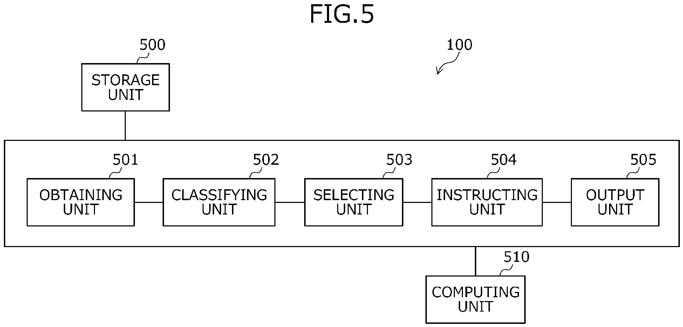

An example of a functional configuration of the information processing device 100 is described with reference to Fig. 5.

Fig. 5 is a block diagram of the example of a functional configuration of the information processing device 100. The information processing device 100 includes a storage unit 500, an obtaining unit 501, a classifying unit 502, a selecting unit 503, an instructing unit 504, and an output unit 505. The information processing device 100 is communicable with a computing unit 510. The computing unit 510 may be included in the information processing device 100.

The storage unit 500 is implemented by, for example, a storage region such as the memory 302 or the recording medium 305 depicted in Fig. 3. Although in the following, a case is described where the storage unit 500 is included in the information processing device 100, this is not limitative. For example, the storage unit 500 may be included in a device different from the information processing device 100 so that the storage content of the storage unit 500 may be referred to by the information processing device 100.

The obtaining unit 501 to the output unit 505 function as an example of a controller. For example, the obtaining unit 501 to the output unit 505 implement functions, for example, by the network I/F 303 or by the CPU 301 executing a program stored in the storage region such as the memory 302 or the recording medium 305 depicted in Fig. 3. The processing results of the functional units are stored, for example, in the storage region such as the memory 302 or the recording medium 305 depicted in Fig. 3.

The storage unit 500 stores various types of information referred to or updated in the processing of the functional units. The storage unit 500 stores, for example, the Green's function expressed by qubits, used when analyzing characteristics of a substance. For example, the storage unit 500 stores the qubit-expressed formula (4) representing the Green's function. For example, the Green's function is set in advance. For example, the Green's function may be obtained by the obtaining unit 501.

The storage unit 500 stores, for example, information enabling identification of quantum circuits corresponding to a specific term. For example, the storage unit 500 stores an index identifying each of the quantum circuits corresponding to the specific term. For example, the information enabling identification of the quantum circuits is set in advance. For example, the information enabling identification of the quantum circuits may be obtained by the obtaining unit 501.

The storage unit 500 stores, for example, information enabling identification of groups in which multiple quantum circuits are classified, for example, such that depending on the characteristics of a substance, quantum circuits corresponding to a same expected value among the quantum circuits are classified into a same group. The characteristics of a substance include, for example, at least any one of characteristics consisting of space-group symmetry, gauge symmetry, particle-hole symmetry, and time-reversal symmetry.

Among the groups are, for example, groups in which two or more quantum circuits are classified. The groups are identified by, for example, the classifying unit 502. The information enabling identification of the quantum circuits is generated by, for example, the classifying unit 502. For example, the information enabling identification of the quantum circuits may be obtained by the obtaining unit 501.

The storage unit 500 stores, for example, information enabling identification of a quantum circuit selected from the groups in which two or more quantum circuits are classified. For example, the storage unit 500 stores indices identifying the quantum circuits. A quantum circuit is selected by, for example, the selecting unit 503. The information enabling identification of a quantum circuit is generated by, for example, the selecting unit 503.

The obtaining unit 501 obtains various types of information for use in processing by the functional units. The obtaining unit 501 stores the obtained various types of information into the storage unit 500 or outputs the information to the functional units. The obtaining unit 501 may output various types of information stored in the storage unit 500 to the functional units. The obtaining unit 501 obtains various types of information, for example, based on a user's operation input. The obtaining unit 501 may receive various types of information, for example, from a device different from the information processing device 100.

For example, the obtaining unit 501 obtains the Green's function. For example, based on a user's operation input, the obtaining unit 501 accepts input of the Green's function and thereby obtains the Green's function. For example, the obtaining unit 501 may receive and obtain the Green's function from another computer. More specifically, when the Green's function is not set in advance, the obtaining unit 501 obtains the Green's function.

The obtaining unit 501 obtains, for example, information enabling identification of multiple quantum circuits corresponding to a specific term. For example, based on a user's operation input, the obtaining unit 501 accepts input of information enabling identification of the quantum circuits and thereby obtains the information enabling identification of the quantum circuits. For example, the obtaining unit 501 may receive and obtain the information enabling identification of the quantum circuits from another computer. More specifically, when the information enabling identification of the quantum circuits is not set in advance, the obtaining unit 501 obtains the information enabling identification of the quantum circuits.

The obtaining unit 501 obtains, for example, information enabling identification of multiple groups. For example, based on a user's operation input, the obtaining unit 501 accepts input of information enabling identification of the groups and thereby obtains the information enabling identification of the groups. For example, the obtaining unit 501 may receive and obtain the information enabling identification of the groups from another computer. More specifically, when the classifying unit 502 does not classify the quantum circuits, the obtaining unit 501 obtains the information enabling identification of the groups.

The obtaining unit 501 obtains, for example, a calculation request requesting calculation of the Green's function. The calculation request may contain, for example, the Green's function and information enabling identification of the quantum circuits. The calculation request may contain, for example, information enabling identification of the groups. For example, based on a user's operation input, the obtaining unit 501 accepts input of a calculation request and thereby obtains the calculation request. For example, the obtaining unit 501 may receive and obtain the calculation request from another computer.

The obtaining unit 501 may receive a start trigger to start the processing of any functional unit. The start trigger is, for example, input of a predetermined user operation. The start trigger may be, for example, receiving predetermined information from another computer. The start trigger may be, for example, output of predetermined information from any functional unit.

The obtaining unit 501 may regard, as the start trigger to start the processing of the classifying unit 502, for example, obtaining information enabling identification of the quantum circuits. The obtaining unit 501 may regard, as the start trigger to start the processing of the selecting unit 503 and the instructing unit 504, for example, obtaining information enabling identification of groups.

The obtaining unit 501 may regard, as the start trigger to start the processing of the classifying unit 502, the selecting unit 503, and the instructing unit 504, for example, obtaining a calculation request containing the Green's function and information enabling identification of the quantum circuits. The obtaining unit 501 may regard, as the start trigger to start the processing of the selecting unit 503 and the instructing unit 504, for example, obtaining a calculation request containing the Green's function, information enabling identification of the quantum circuits, and information enabling identification of the groups.

The classifying unit 502 classifies the quantum circuits into groups. The classifying unit 502 classifies the quantum circuits into the groups, for example, such that depending on the characteristics of a substance, quantum circuits corresponding to a same expected value among the quantum circuits are classified into a same group. This enables the classifying unit 502 to identify the groups. The classifying unit 502 may obtain a policy for reducing the processing burden and the processing time in calculating the Green's function.

Depending on the characteristics of a substance, the classifying unit 502 identifies a quantum circuit whose expected value is zero among the quantum circuits. This enables the classifying unit 502 to identify quantum circuits whose expected values are not measured when calculating the Green's function and thereby, obtain a policy for reducing the processing burden and the processing time in calculating the Green's function.

The selecting unit 503 selects a quantum circuit from each of one or more groups into which two or more quantum circuits are classified, among the groups.

For example, for each of the one or more groups into which two or more quantum circuits are classified among the groups, the selecting unit 503 identifies the number of Pauli gates that form each of the two or more quantum circuits classified into the group. For example, based on the identified number of Pauli gates, for each of the one or more groups, the selecting unit 503 selects from the group, a quantum circuit having a relatively small number of Pauli gates identified.

Thus, for each of the one or more groups, the selecting unit 503 may select a quantum circuit whose expected value is actually to be measured and may enable identification of another quantum circuit different from the selected quantum circuit in the group and whose expected value needs not be measured. For each of the one or more groups, the selecting unit 503 may select a quantum circuit having a relatively small number of Pauli gates as the quantum circuit whose expected value is to be actually measured. As a result, the selecting unit 503 may achieve reduction in processing burden and processing time imposed when actually measuring the expected value for each of one or more groups.

For example, the selecting unit 503 may select from among the groups, a quantum circuit at random from each of the one or more groups into which two or more quantum circuits are classified. Thus, for each of the one or more groups, the selecting unit 503 may select a quantum circuit whose expected value is actually to be measured and may enable identification of another quantum circuit different from the selected quantum circuit in the group and whose expected value needs not be measured.

For example, based on a user's operation input, the selecting unit 503 may receive selection of a quantum circuit from each of the one or more groups into which two or more quantum circuits are classified, among the groups. As a result, for each of the one or more groups, the selecting unit 503 may select a quantum circuit whose expected value is actually to be measured, to thereby enable identification of another quantum circuit different from the selected quantum circuit in the group and whose expected value needs not be measured,.

The instructing unit 504 controls the computing unit 510. The instructing unit 504 generates a calculation request requesting calculation of the Green's function and enabling identification of a quantum circuit selected by the selecting unit 503 from each of the one or more groups into which two or more quantum circuits are classified, among the groups. The calculation request may contain, for example, the Green's function and information enabling identification of the groups and the quantum circuits. The calculation request may include information enabling identification of a quantum circuit having an expected value of zero. For example, the instructing unit 504 transmits the generated calculation request to the computing unit 510 to thereby control the computing unit 510.

For example, the instructing unit 504 controls the computing unit 510 so as to measure, for each of the one or more groups into which two or more quantum circuits are classified, the expected value represented by a quantum circuit selected by the selecting unit 503 from the group. For example, when there are one or more groups in which one quantum circuit is classified, the instructing unit 504 controls the computing unit 510 so as to measure, for each of the one or more groups, the expected value represented by the one quantum circuit belonging to the group.

For example, the instructing unit 504 controls the computing unit 510 so as to identify, for each of the one or more groups into which two or more quantum circuits are classified, another quantum circuit different from a quantum circuit selected by the selecting unit 503, in the group. For example, the instructing unit 504 controls the computing unit 510 so as to omit, for each of the one or more groups into which two or more quantum circuits are classified, processing of measuring the expected value represented by the identified other quantum circuit in the group. The instructing unit 504 may thus reduce the processing burden and the processing time imposed on the computing unit 510 in calculating the Green's function.

For example, the instructing unit 504 controls the computing unit 510 so as to omit the processing of measuring the expected value of a quantum circuit whose expected value is ensured to be zero by the symmetry among the quantum circuits. This enables the instructing unit 504 to reduce the processing burden and the processing time imposed on the computing unit 510 when calculating the Green's function.

The instructing unit 504 receives the result of calculation of the Green's function from the computing unit 510.

The output unit 505 outputs the result of processing of at least any one of the functional units. Examples of the output format include: display onto a display; output to a printer for printout, transmission to an external device by the network I/F 303; and storage into a storage region such as the memory 302 or the recording medium 305. This allows the output unit 505 to notify the user of the processing result of at least any one of the functional units, thereby achieving improvement in convenience of the information processing device 100.

The output unit 505 outputs, for example, multiple groups identified by the classifying unit 502. For example, the output unit 505 outputs the groups in such a manner as to enable reference by the user. For example, the output unit 505 may transmit the groups to another computer. This enables the output unit 505 to make the groups available externally.

The output unit 505 outputs, for example, information enabling identification of a quantum circuit selected by the selecting unit 503 from each of the one or more groups into which two or more quantum circuits are classified. For example, the output unit 505 outputs the information enabling identification of a quantum circuit selected by the selecting unit 503, in such a manner as to enable reference by the user. For example, the output unit 505 may transmit the information enabling identification of a quantum circuit selected by the selecting unit 503, to another computer. Thus, the output unit 505 may make the information enabling identification of a quantum circuit selected by the selecting unit 503 available externally.

The output unit 505 outputs, for example, the result of calculation of the Green's function received by the instructing unit 504. For example, the output unit 505 outputs the calculation result of the Green's function in such a manner as to enable reference by the user. For example, the output unit 505 may transmit the calculation result to another computer. Thus, the output unit 505 may make the calculation result of the Green's function available externally.

The computing unit 510 receives a calculation request. Based on the calculation request, the computing unit 510 identifies the Green's function, quantum circuits corresponding to a specific term, and groups. Based on the calculation request, for each of the one or more groups into which two or more quantum circuits are classified, the computing unit 510 identifies a quantum circuit selected from the group and another quantum circuit different from the selected quantum circuit.

For each of the one or more groups into which two or more quantum circuits are classified, the computing unit 510 measures an expected value represented by the quantum circuit selected from the group. When among the groups, there are one or more groups into which one quantum circuit is classified, the computing unit 510 measures, for each of the one or more groups, an expected value represented by one quantum circuit belonging to the group.

For each of the one or more groups into which two or more quantum circuits are classified, the computing unit 510 omits the processing of measuring the expected value represented by the identified other quantum circuit in the each group. For example, for each of the one or more groups into which two or more quantum circuits are classified, the computing unit 510 uses the measured expected value represented by any quantum circuit in the group as expected values represented by the identified other quantum circuits.

The computing unit 510 omits the processing of measuring the expected value represented by a quantum circuit whose expected value is zero among the quantum circuits. The computing unit 510 uses zero as an expected value represented by a quantum circuit whose expected value is zero among the quantum circuits.

Thus, the computing unit 510 may make the expected value represented by each of the quantum circuits available. For each of the one or more groups into which two or more quantum circuits are classified, the computing unit 510 may omit the processing of measuring the expected value represented by the identified other quantum circuits in the group, achieving reduction in processing amount and processing time.

The computing unit 510 calculates the Green's function using the expected value represented by each of the quantum circuits corresponding to a specific term that forms the Green's function. As a result, the computing unit 510 may calculate the Green's function. The computing unit 510 may reduce the processing burden imposed and the processing time taken when calculating the Green's function.

Although here, a case has been described where the information processing device 100 includes the obtaining unit 501, the classifying unit 502, the selecting unit 503, the instructing unit 504, and the output unit 505, this is not limitative. For example, the information processing device 100 may omit any of the functional units. For example, the information processing device 100 may omit the classifying unit 502.

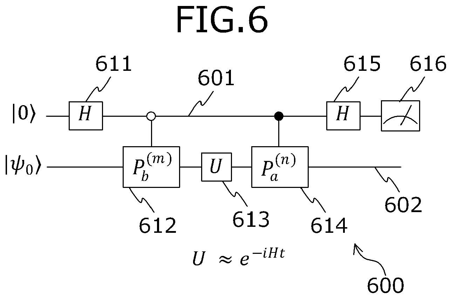

An operation example of the information processing device 100 is described with reference to Figs. 6 to 11. In the operation example, the information processing device 100 controls the quantum computer 201 so as to calculate a Green's function that is defined for a fermionic Hamiltonian H. For example, the Green's function is expressed by the formula (1).

Here, $\theta(t)$ is a step function. $|\psi_0\rangle$ is a ground state of the Hamiltonian H. {A,B}=AB+BA is an anticommutator for operators A and B. $c_a$ and $c_b\dagger$ are an annihilation operator and a generation operator, respectively, of a fermion. "a" and "b" are subscripts indicating a fermion mode. In the case of calculating the Green's function, the quantum computer 201 handles $c_a$ and $c_b\dagger$ in Pauli matrix representation as depicted in formulae (6) and (7) below.

$$c_a \mapsto \frac{1}{2}\left(P_a^{(1)} + iP_a^{(2)}\right) \tag{6}$$

$$c_a^\dagger \mapsto \frac{1}{2}\left(P_a^{(1)} - iP_a^{(2)}\right) \tag{7}$$

Here, $P_a^{(n)}$ (n=1,2) represents a tensor product of the Pauli matrix. The formulae (6) and (7) are specific examples of the formula (3), and Jordan-Wigner transform as a standard technique may be expressed in the format of the formula (3). Accordingly, the formula (1) may be rewritten as formula (8) below. $K_{a,b}^{(n,m)}(t)$ in the formula (8) is expressed by formula (9) below.

$$G_{a,b}^R(t) = -\frac{i}{2}\theta(t)\left[(K_{a,b}^{(1,1)}(t) + K_{a,b}^{(2,2)}(t)) - i(K_{a,b}^{(1,2)}(t) - K_{a,b}^{(2,1)}(t))\right]$$

$$\tag{8}$$

$$K_{a,b}^{(n,m)}(t) = Re\langle\psi_0|e^{iHt}P_a^{(n)}e^{-iHt}P_b^{(m)}|\psi_0\rangle \tag{9}$$

Where, $K_{a,b}^{(n,m)}(t)$ corresponds to an expected value represented by a quantum circuit 600 that is described later in Fig. 6. The Green's function may therefore be calculated by identifying an expected value represented by each of multiple quantum circuits 600 corresponding to all combinations of the subscripts (a,b) and (n,m) of $K_{a,b}^{(n,m)}(t)$.

Here, combinations of (n,m) are (1,1), (1,2), (2,1), and (2,2). Accordingly, when the subscript a takes M different values, the total combinations of the subscripts (a,b) and (n,m) is $4M^2$. The Green's function may therefore be calculated by identifying an expected value represented by each of $4M^2$ quantum circuits 600.

Here, an example of the quantum circuit 600 is described with reference to Fig. 6.

Fig. 6 is an explanatory view of an example of the quantum circuit 600. In Fig. 6, a top line 601 of the quantum circuit 600 corresponds to an auxiliary qubit. A bottom line 602 of the quantum circuit 600 corresponds to a qubit representing a system. Reference numerals 611, 612, 613, 614, and 615 denote quantum gates. Reference numeral 616 denotes measuring an expected value for the auxiliary qubit. The expected value for the auxiliary qubit is $Re\langle\psi_0|U\dagger P_a^{(n)}UP_b^{(m)}|\psi_0\rangle$. Thus, if $U\approx e^{-iHt}$, the expected value for the auxiliary qubit is an approximation of $K_{a,b}^{(n,m)}(t)$.

Here, the information processing device 100 achieves reduction in processing burden and processing time in calculating the Green's function, by setting a quantum circuit 600 whose expected value is to be measured and a quantum circuit 600 whose expected value needs not be measured, among the quantum circuits 600.

For example, the information processing device 100 classifies two or more quantum circuits 600 corresponding to a same expected value into a same group, depending on the characteristics of a substance. For example, the information processing device 100 selects any quantum circuit 600 in a group and sets the selected quantum circuit 600 as a quantum circuit 600 whose expected value is to be measured. For example, the information processing device 100 selects another quantum circuit 600 different from the selected quantum circuit 600 in the group and sets the selected other quantum circuit 600 as a quantum circuit 600 whose expected value needs not be measured.

The characteristics of a substance include, for example, at least any one of characteristics consisting of space-group symmetry, gauge symmetry, particle-hole symmetry, and time-reversal symmetry. The space-group symmetry includes, for example, space symmetry and translation symmetry.

The space symmetry is symmetry on a space operation of a crystal or molecular structure of a substance. The space operation is, for example, rotation, reflection, or reversal. The crystal structure can be classified using, for example, 230 types of space groups.

The translation symmetry is symmetry representing periodicity of the crystal structure in solid crystals of a substance.

The gauge symmetry corresponds to the particle number conservation law. The gauge symmetry is symmetry representing that the total number of electrons remains unchanged. The gauge symmetry is present, for example, when a substance is in a stable state of equilibrium.

The particle-hole symmetry is symmetry representing that electrons and holes are equal in number. The particle-hole symmetry is present, for example, when the substance is a superconductor or a half-filling solid system.

The time-reversal symmetry is a symmetry related to an electron spin reversal operation. The time-reversal symmetry is present, for example, when an external magnetic field, spontaneous magnetization, etc., are absent for the substance.

The information processing device 100 may receive, for example, instruction information indicating which characteristic is established among the characteristics of a substance and designating which characteristic is to be considered among the characteristics of the substance. For example, based on the instruction information, the information processing device 100 classifies two or more quantum circuits corresponding to a same expected value into a same group, depending on the designated characteristic of the substance, and sets a quantum circuit 600 whose expected value is to be measured and a quantum circuit 600 whose expected value needs not be measured.

The instruction information includes, for example, a first parameter related to the space symmetry. The first parameter is, for example, any value from 0 to 230. The first parameter indicates that, for example, when the value is 0, the space symmetry is not considered. The first parameter indicates that, for example, when the value is any value from 1 to 230, the space symmetry is considered in a space group corresponding to the value. The space group is an aggregate of space operations, expressed as a mathematical set. The crystal structures of substances existing in the natural world may each be classified into any one of 230 types of space groups.

The instruction information includes, for example, a second parameter related to the translation symmetry. The second parameter is, for example, a flag indicating whether the translation symmetry is to be considered. For example, when true, the flag is a periodic boundary condition and indicates that the translation symmetry is established and therefore considered. For example, when false, the flag is an open boundary condition and indicates that the translation symmetry is not established and therefore not considered.

The instruction information includes, for example, a third parameter related to the gauge symmetry. The third parameter is, for example, a flag indicating whether the gauge symmetry is to be considered. For example, when true, the flag indicates that the particle number holds and that the gauge symmetry is established and therefore considered. For example, when false, the flag indicates that the particle number does not hold and that the gauge symmetry is not established and therefore not considered.

The instruction information includes, for example, a fourth parameter related to the particle-hole symmetry. The fourth parameter is, for example, a flag indicating whether the particle-hole symmetry is to be considered. For example, when true, the flag indicates that the substance is a superconductor, a half-filling, or the like and that the particle-hole symmetry is established and therefore considered. For example, when false, the flag indicates that the particle-hole symmetry is not established and therefore not considered.

The instruction information includes, for example, a fifth parameter related to the time-reversal symmetry. The fifth parameter is, for example, a flag indicating whether the time-reversal symmetry is to be considered. For example, when true, the flag indicates that the particle-hole symmetry is established and therefore considered. For example, when false, the flag indicates that the substance is under a magnetic field or is a magnetic body and that the time-reversal symmetry is not established and therefore not considered.

A specific example is described where, depending on the gauge symmetry, the information processing device 100 classifies two or more quantum circuits 600 corresponding to a same expected value into a same group. If the Hamiltonian H holds U(1) gauge symmetry, then formula (10) below holds.

$$\langle \psi_0 | e^{iHt} c_a e^{-iHt} c_b | \psi_0 \rangle = \langle \psi_0 | e^{iHt} c_a^\dagger e^{-iHt} c_b^\dagger | \psi_0 \rangle = 0 \tag{10}$$

Based on the formulae (6) and (7), $K_{a,b}^{(n,m)}(t)$ of the formula (9) may be rewritten, by fermionic representation, as a combination of formulae (11), (12), (13,) and (14) which follow.

$$K_{a,b}^{(1,1)}(t) = Re\langle \psi_0 | e^{iHt}(c_a + c_a^\dagger)e^{-iHt}(c_b + c_b^\dagger) | \psi_0 \rangle \tag{11}$$

$$K_{a,b}^{(2,2)}(t) = -Re\langle \psi_0 | e^{iHt}(c_a - c_a^\dagger)e^{-iHt}(c_b - c_b^\dagger) | \psi_0 \rangle \tag{12}$$

$$K_{a,b}^{(1,2)}(t) = -iRe\langle\psi_0|e^{iHt}(c_a + c_a^\dagger)e^{-iHt}(c_b - c_b^\dagger)|\psi_0\rangle \quad (13)$$

$$K_{a,b}^{(2,1)}(t) = -iRe\langle\psi_0|e^{iHt}(c_a - c_a^\dagger)e^{-iHt}(c_b + c_b^\dagger)|\psi_0\rangle \quad (14)$$

Application of the formula (10) to the formulae (11) to (14) leads formulae (15) and (16) which follow.

$$K_{a,b}^{(1,1)}(t) = K_{a,b}^{(2,2)}(t) = Re\langle\psi_0|e^{iHt}c_a e^{-iHt}c_b^\dagger + e^{iHt}c_a^\dagger e^{-iHt}c_b|\psi_0\rangle \quad (15)$$

$$K_{a,b}^{(1,2)}(t) = -K_{a,b}^{(2,1)}(t) = Re\langle\psi_0|e^{iHt}c_a e^{-iHt}c_b^\dagger - e^{iHt}c_a^\dagger e^{-iHt}c_b|\psi_0\rangle \quad (16)$$

Thus, the information processing device 100 classifies a quantum circuit 600 representing $K_{a,b}^{(1,1)}(t)$ and a quantum circuit 600 representing $K_{a,b}^{(2,2)}(t)$ into a same group. The information processing device 100 sets, as a quantum circuit 600 whose expected value needs not be measured, either one of the quantum circuit 600 representing $K_{a,b}^{(1,1)}(t)$ and the quantum circuit 600 representing $K_{a,b}^{(2,2)}(t)$ that have been classified into a same group.

The information processing device 100 classifies a quantum circuit 600 representing $K_{a,b}^{(1,2)}(t)$ and a quantum circuit 600 representing $K_{a,b}^{(2,1)}(t)$ into a same group. The information processing device 100 sets, as a quantum circuit 600 whose expected value needs not be measured, either one of the quantum circuit 600 representing $K_{a,b}^{(1,2)}(t)$ and the quantum circuit 600 representing $K_{a,b}^{(2,1)}(t)$ that have been classified into a same group..

Next, a specific example is described where, for example, depending on the time-reversal symmetry, the information processing device 100 classifies two or more quantum circuits 600 corresponding to a same expected value into a same group. Formula (17) below represents that the Hamiltonian H is invariable under a certain target operation R ^. Here, for the sake of convenience, a symbol obtained by imparting ^ to R may be written as "R ^".

$$\left[H, \widehat{R}\right] = 0 \qquad\qquad (17)$$

Application of the formula (17) to the formulae (15) to (16) leads formula (18) below.

$$K_{a,b}^{(n,m)}(t) = Re\langle\psi_0|e^{iHt}(\widehat{R}c_a\widehat{R}^{-1})e^{-iHt}(\widehat{R}c_b^\dagger\widehat{R}^{-1})|\psi_0\rangle +$$

$$2\left(\frac{1}{2}-\delta_{mn}\right)Re\langle\psi_0|e^{iHt}(\widehat{R}c_a^\dagger\widehat{R}^{-1})e^{-iHt}(\widehat{R}c_b\widehat{R}^{-1})|\psi_0\rangle \qquad (18)$$

Here, $\delta_{nm}$ is Kronecker's delta. A specific example is described where, depending on the characteristics of a substrate, the information processing device 100 classifies, based on the formula (18), two or more quantum circuits 600 corresponding to a same expected value into a same group.

In the description, it is assumed that a substrate is represented by an N-site lattice model that is defined by the periodic boundary condition. The fermion mode is assumed to be designated by a site number j (=1,2,...,N) and a spin $\sigma$ (=↑,↓). Accordingly, a=jσ(=1t,2t,...,Nt,1↓,2↓,...,N↓). The total of the subscripts (a,b)=(jσ,j'σ') is $(2N)^2=4N^2$.

Here, the effect of a time reversal operation Θ^ on a fermion operator is defined by formulae (19) and (20) below.

$$\widehat{\Theta}c_{j\uparrow}^{(\dagger)}\widehat{\Theta}^{-1} = c_{j\downarrow}^{(\dagger)} \qquad\qquad (19)$$

$$\widehat{\Theta}c_{j\downarrow}^{(\dagger)}\widehat{\Theta}^{-1} = -c_{j\uparrow}^{(\dagger)} \qquad\qquad (20)$$

In cases where the time-reversal symmetry holds, [H, Θ^]=0 is established. Thus, if R^=Θ^ in the formula (18), formulae (21) and (22) below hold based on the formulae (19) and (20).

$$K_{j\uparrow,j'\uparrow}^{(n,m)}(t) = K_{j\downarrow,j'\downarrow}^{(n,m)}(t) \qquad\qquad (21)$$

$$K_{j\uparrow,j'\uparrow}^{(n,m)}(t) = -K_{j\downarrow,j'\downarrow}^{(n,m)}(t) \tag{22}$$

Thus, the information processing device 100 classifies a quantum circuit 600 representing $K_{j\uparrow,j'\uparrow}^{(n,m)}(t)$ and a quantum circuit 600 representing $K_{j\downarrow,j'\downarrow}^{(n,m)}(t)$ into a same group. The information processing device 100 sets, as a quantum circuit 600 whose expected value needs not be measured, either one of the quantum circuit 600 representing $K_{j\uparrow,j'\uparrow}^{(n,m)}(t)$ and the quantum circuit 600 representing $K_{j\downarrow,j'\downarrow}^{(n,m)}(t)$ that have been classified into a same group..

The information processing device 100 classifies a quantum circuit 600 representing $K_{j\uparrow,j'\downarrow}^{(n,m)}(t)$ and a quantum circuit 600 representing $K_{j\downarrow,j'\uparrow}^{(n,m)}(t)$ into a same group. The information processing device 100 sets, as a quantum circuit 600 whose expected value needs not be measured, either one of the quantum circuit 600 representing $K_{j\uparrow,j'\downarrow}^{(n,m)}(t)$ and the quantum circuit 600 representing $K_{j\downarrow,j'\uparrow}^{(n,m)}(t)$ that have been classified into a same group..

A specific example is described where, for example, depending on the space-group symmetry, the information processing device 100 classifies two or more quantum circuits 600 corresponding to a same expected value into a same group. The space-group symmetry includes, for example, space symmetry and translation symmetry. The space-group symmetry is, for example, symmetry representing invariance to space operations of crystal structure.

The space operation includes rotation, reflection, reversal, and translation. The space group is an aggregate of space operations, expressed as a mathematical set. The crystal structures of substances existing in the natural world may each be classified into any one of 230 types of space groups. The effect of a symmetry operation $g_k{}^\wedge$ belonging to a space group G is expressed by formula (23) below.

$$\hat{g}_k c_{j\sigma}^{(\dagger)} \hat{g}_k^{-1} = -c_{\pi_k(j)\sigma}^{(\dagger)} \tag{23}$$

Here, $\pi_k$ is expressed by formula (24) below. The formula (24) represents a transformation rule for a site index j implemented by the symmetry operation $g_k{}^\wedge$ belonging to the space group G.

$$\pi_k = \begin{pmatrix} 1 & 2 & \cdots & N \\ \pi_k(1) & \pi_k(2) & \cdots & \pi_k(N) \end{pmatrix} \tag{24}$$

In cases where the crystal structure of a system belongs to the space group G, the Hamiltonian H is invariable under a symmetry operation $g_k{}^\wedge \in G$. In other words, $[H, g_k{}^\wedge]=0$. Thus, if $R^\wedge = g_k{}^\wedge$ in the formula (18), the formula (23) leads formula (25) below.

$$K_{j\sigma,j'\sigma'}^{(n,m)}(t) = -K_{\pi_k(j)\sigma,\pi_k(j')\sigma'}^{(n,m)}(t) \tag{25}$$

Thus, the information processing device 100 sets, as a quantum circuit 600 whose expected value needs not be measured, either one of the quantum circuit 600 representing $K_{j\sigma,j'\sigma'}^{(n,m)}(t)$ and the quantum circuit 600 representing $K_{\pi_{k(j)}\sigma,\pi_{k(j')}\sigma'}^{(n,m)}(t)$.

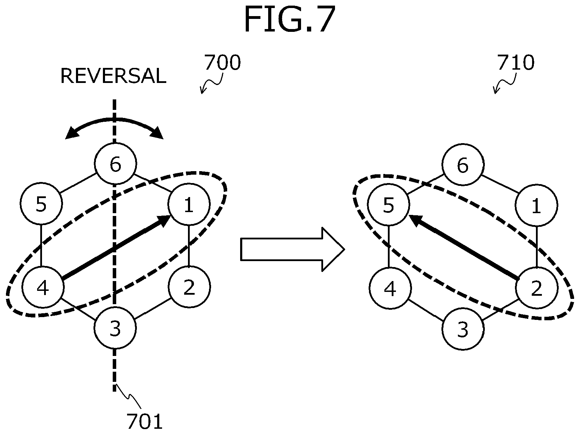

For example, the symmetry operation on a two-dimensional square lattice of site number $N=L^2$ may be 4-fold rotation operation, vertical reflection operation, horizontal reflection operation, translation operation, or the like. Considering the formula (25) in relation to the target operation, the number of combinations (j,j') of independent site indices having different values of $K_{j\sigma,j'\sigma'}^{(n,m)}(t)$ is $(^\wedge N+2)(^\wedge N+4)/8$. Here, with reference to Fig. 7, a specific example is described where, depending on the space symmetry, the information processing device 100 classifies two or more quantum circuits 600 corresponding to a same expected value into a same group.

Fig. 7 is an explanatory view of an example of classifying two or more quantum circuits 600 corresponding to a same expected value into a same group. Fig. 7 depicts a structure 700 of a substrate. The structure 700 has a similar structure 710 even when reversed left and right around an axis 701. Accordingly, the expected value related to a relationship between electrons 4 and 1 is equal to the expected value related to a relationship between electrons 2 and 5. Here, for the sake of convenience, for example, an electron on a site 1 is referred to as electron 1. Thus, Green's function $G_{14}^R(t)$=Green's function $G_{52}^R(t)$.

Thus, the information processing device 100 sets, as a quantum circuit 600 whose expected value needs not be measured, a quantum circuit 600 representing either one of the two expected values, i.e., the expected value related to the relationship between the electrons 4 and 1 and the expected value related to the relationship between the electrons 2 and 5. By measuring either one of the two expected values, the information processing device 100, without measuring the other expected value, may specify that the two expected values are available for use.

A specific example is described where, for example, depending on the particle-hole symmetry, the information processing device 100 classifies two or more quantum circuits 600 corresponding to a same expected value into a same group. The particle-hole symmetry is symmetry representing that electrons and holes are equal in number. In cases where the total number of electrons is equal to the number of sites in the site-N lattice model, the particle-hole symmetry holds. The definition of the particle-hole symmetry depends on the existence of the sublattice degree of freedom.

Here, for example, in a lattice model composed of two sublattices A and B, a particle-hole transform $\hat{\Xi}$ is defined by formulae (26) and (27) below.

$$\hat{\Xi} c_{j\sigma} \hat{\Xi}^{-1} = \eta_j c_{j\sigma}^{\dagger} \qquad (26)$$

$$\eta_j = \begin{cases} +1 & (j \in A) \\ -1 & (j \in B) \end{cases} \qquad (27)$$

In cases where the particle-hole symmetry holds, $[H, \hat{\Xi}]=0$. Thus, if $R^{\wedge}=\Xi^{\wedge}$ in the formula (18), the formula (26) leads formula (28) below.

$$K_{j\sigma,j'\sigma'}^{(n,m)}(t) = \begin{cases} \left(1 + \eta_j \eta_{j'}\right) Re \langle \psi_0 \left| e^{iHt} c_{j\sigma} e^{-iHt} c_{j'\sigma'}^{\dagger} \right| \psi_0 \rangle \ (n = m) \\ i\left(1 - \eta_j \eta_{j'}\right) Re \langle \psi_0 \left| e^{iHt} c_{j\sigma} e^{-iHt} c_{j'\sigma'}^{\dagger} \right| \psi_0 \rangle \ (n \neq m) \end{cases}$$

$$(28)$$

Therefore, in cases where the sites j and j' belong to a same sublattice, as to $(q_j q_{j'}=+1)$, the diagonal component $K_{j\sigma,j'\sigma'}^{(n,n)}$ for (n,m) is non-zero, while the off-diagonal component is zero. On the other hand, in cases where the sites j and j' belong to different sublattices, as to $(\eta_j \eta_{j'}=-1)$, the diagonal component $K_{j\sigma,j'\sigma'}^{(n,m)}(n \neq m)$ for (n,m) is non-zero, while the other diagonal components are zero. Thus, the information processing device 100 sets, as a quantum circuit 600 whose expected value needs not be measured, a quantum circuit 600 representing $K_{j\sigma,j'\sigma'}^{(n,m)}$ of zero in a system where the particle-hole symmetry holds.

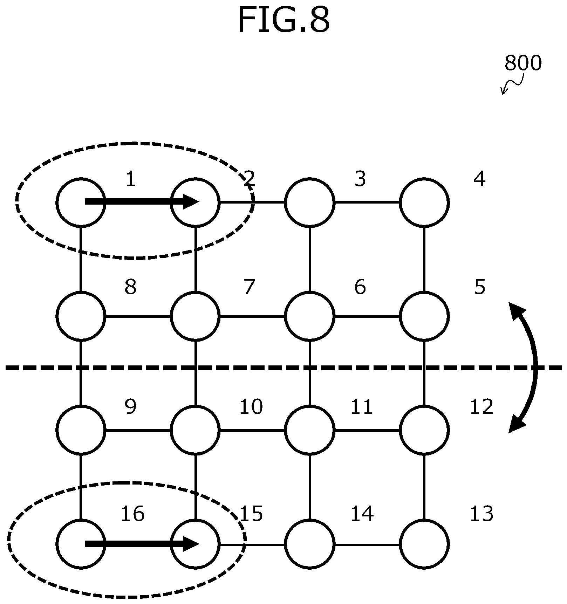

Referring then to Fig. 8, An example is described where the information processing device 100 selects any quantum circuit 600 from a group and sets the selected quantum circuit 600 as a quantum circuit 600 whose expected value is to be measured.

Fig. 8 is an explanatory view of an example of selecting a quantum circuit 600 from a group. Here, two or more quantum circuits 600 belonging to a group have a nature of differing in processing burden and processing time when carrying out measurement of their respective expected values, in spite of corresponding to the same expected value. For example, there is a nature of differing in the number of Pauli gates that form each of the two or more quantum circuits 600 belonging to the group.

For example, the Pauli gates forming a quantum circuit 600 are elements attributable to a generation operator and an annihilation operator of a fermion (electron). The fermion generation operator and annihilation operator have a characteristic (anti-commutativity) that the sign is reversed when the order is changed. Thus, when the fermion generation operator and annihilation operator are represented in qubits, the number of Pauli gates differs depending on the index.

For example, the wave function representing a state in which N electrons exist is expressed by formula (29) below. Application of an annihilation operator $c_i$ to the formula (29) below leads formula (30) also below. For example, since the annihilation operator $c_i$ is applied to annihilate an i-th electron, the annihilation operator $c_i$ is substituted with 1 to (i-1)th operators, resulting in multiplication by the number of substitutions (-1).

$$|\Psi\rangle = \left(c_1^{\dagger}\right) \cdots \left(c_{i-1}^{\dagger}\right)\left(c_i^{\dagger}\right)\left(c_{i+1}^{\dagger}\right) \cdots \left(c_N^{\dagger}\right)|0\rangle \qquad (29)$$

$$c_i|\Psi\rangle = c_i\left(c_1^{\dagger}\right) \cdots \left(c_{i-1}^{\dagger}\right)\left(c_i^{\dagger}\right)\left(c_{i+1}^{\dagger}\right) \cdots \left(c_N^{\dagger}\right)|0\rangle = (-1)^{i-1}\left(c_1^{\dagger}\right) \cdots \left(c_{i-1}^{\dagger}\right)\left(c_{i+1}^{\dagger}\right) \cdots \left(c_N^{\dagger}\right)|0\rangle$$

$$(30)$$

In cases where $(-1)^{i-1}$ is qubit-represented by Jordan-Wigner transform, a Pauli gate given by formula (31) below is used. As a result, the number of Pauli gates forming a quantum circuit 600 is different depending on the value of the index i.

$$Z_1 \otimes Z_2 \otimes \cdots \otimes Z_{i-1} \qquad (31)$$

In an example depicted in Fig. 8, for example, in a 4×4 lattice model depicted in Fig. 8, one expected value corresponding to a pair (1,2) of atoms 1 and 2 is expressed by formula (32) below. Thus, 3 is the number of Pauli gates of a quantum circuit 600 representing expected values corresponding to the pair (1,2) of the atoms 1 and 2.

$$Re\langle\psi_0|U^\dagger(X_1)U(Y_2Z_1)|\psi_0\rangle \qquad (32)$$

For example, in the 4×4 lattice model depicted in Fig. 8, one of expected values corresponding to a pair (16,15) of atoms 16 and 15 is expressed by formula (33) below. Thus, 31 is the number of Pauli gates of a quantum circuit 600 representing expected values corresponding to the pair (16,15) of the atoms 16 and 15.

$$e\langle\psi_0|U^\dagger(X_{16}Z_{15}\cdots Z_1)U(Y_{15}Z_{14}\cdots Z_1)|\psi_0\rangle \qquad (33)$$

Thus, preferably, as a quantum circuit 600 whose expected value is to be measured, the information processing device 100 may select a quantum circuit 600 having the least number of Pauli gates among two or more quantum circuits 600 belonging to a group. In other words, preferably, as a quantum circuit 600 whose expected value needs not be measured, the information processing device 100 may select a quantum circuit 600 having a relatively large number of Pauli gates, among the two or more quantum circuits 600 belonging to the group.

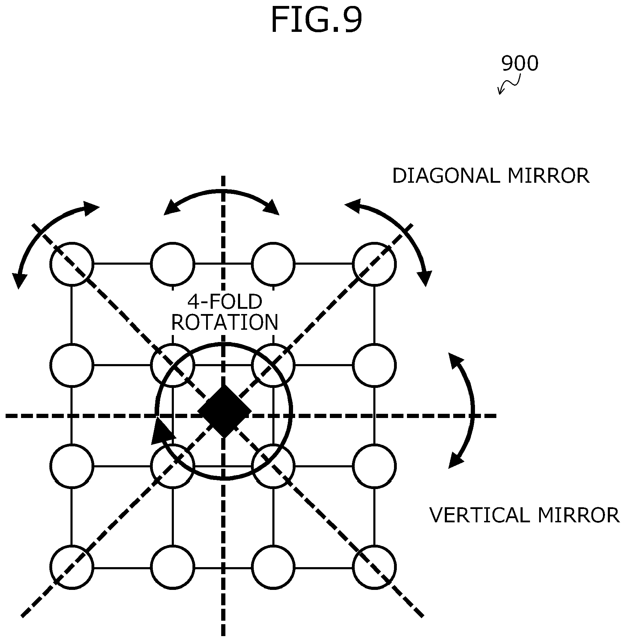

An example of an advantageous effect achieved by the information processing device 100 is described with reference to Figs. 9 to 11.

Figs. 9, 10, and 11 are explanatory views of an example of the advantageous effect. In the examples depicted Figs. 9 to 11, a case is described where the information processing device 100 reduces the processing time and the processing burden imposed when calculating the Green's function of a Hubbard model 900 depicted in Fig. 9. The Hubbard model 900 is a model describing, in quantum theory, the behavior of electrons in a solid that has strong electronic correlation.

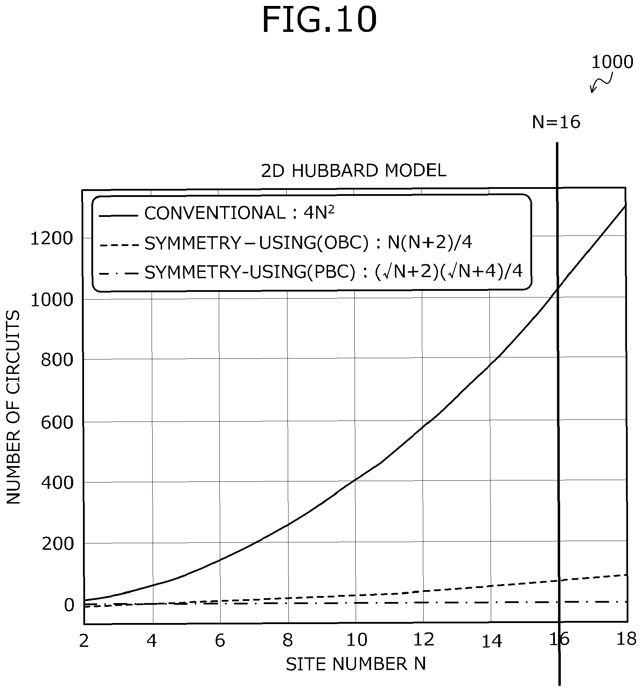

In this case, considering the space symmetry, the gauge symmetry, the translation symmetry, and the particle-hole symmetry, the information processing device 100 selects a quantum circuit 600 whose expected value is to be measured. The space symmetry is related to a space group that depends on the shape of a lattice, and has symmetry operations including a 4-fold rotation, a diagonal mirror, and a vertical mirror depicted in Fig. 9. The translation symmetry is established in a case of a periodic boundary condition. The particle-hole symmetry is established in a case of half-filling. Jordan-Wigner transform is used for qubit representation of the Green's function. Fig. 10 is described.

In Fig. 10, $N=L^2$ is assumed, where N denotes the number of sites of the Hubbard model 900. Let N be an even number. Here, the number of measurements is $4N^2$ in the conventional technique of measuring the expected values represented by all of the quantum circuits 600 without consideration of a similarly that a substance has.

On the other hand, in the case of the open boundary condition (OBC), the number of measurements is $N(N+2)/4$ in the novel technique where the information processing device 100 takes the symmetry of the substance into consideration and sets a quantum circuit 600 whose expected value needs not be measured. Furthermore, in the case of half-filling, the number of measurements is $N(N+2)/8$ in the novel technique.

In the case of periodic boundary condition (PBC), the number of measurements is $(^\wedge N+2)(^\wedge N+4)/4$ in the novel technique where the information processing device 100 takes the symmetry of the substance into consideration and sets a quantum circuit 600 whose expected value needs not be measured. Furthermore, in the case of half-filling, the number of measurements is $(^\wedge N+2)(^\wedge N+4)/8$ in the novel technique.

A graph 1000 depicted Fig. 10 shows the number of quantum circuits 600 whose expected values are to be measured in the conventional technique (conventional). The graph 1000 in Fig. 10 shows the number of quantum circuits 600 whose expected values are to be measured in the novel technique (symmetry-using (OBC)) in the case of the open boundary condition (OBC). The graph 1000 in Fig. 10 shows the number of quantum circuits 600 whose expected values are to be measured in the novel technique (symmetry-using (PBC)) in the case of the periodic boundary condition (PBC).

For example, for the Hubbard model 900 of 4×4 site number N=16, the number of quantum circuits 600 whose expected values are to be measured is 1024 in the conventional technique. For example, for the Hubbard model 900 of 4×4 site number N=16, the number of quantum circuits 600 whose expected values are to be measured is 72 in the novel technique (symmetry-using (OBC)). For example, for the Hubbard model 900 of 4×4 site number N=16, the number of quantum circuits 600 whose expected values are to be measured is 12 in the novel technique (symmetry-using (PBC)).

In this manner, in the case of the open boundary condition (OBC), the information processing device 100 may reduce the number of quantum circuits 600 whose expected values are to be measured, to approx. 1/14 as compared with the conventional technique. Thus, the information processing device 100 may achieve reduction in processing burden and processing time imposed when calculating the Green's function, as compared with the conventional technique.

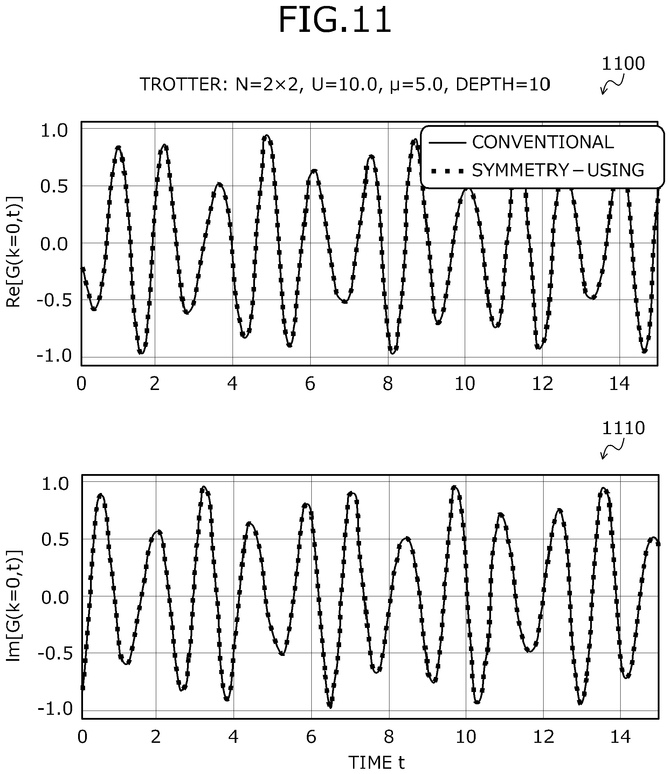

In the same manner, in the case of the periodic boundary condition (PBC), the information processing device 100 may reduce the number of quantum circuits 600 whose expected values are to be measured, to approx. 1/85 as compared with the conventional technique. Thus, the information processing device 100 may achieve reduction in processing burden and processing time imposed when calculating the Green's function, as compared with the conventional technique. Fig. 11 is described.

In an example depicted in Fig. 11, it is assumed that the quantum computer 201 calculates a Green's function for the Hubbard model 900 of $2 \times 2$ site number N=4, based on the quantum circuits 600 whose expected values are to be measured, set by the information processing device 100. Let the ground state $|\psi_0>$ be exact solution. The time evolution operator U is assumed to be approximated by Trotter decomposition.

A graph 1100 depicted in Fig. 11 depicts, by a dotted line, a real part obtained when the quantum computer 201 calculates the Green's function, based on the quantum circuits 600 whose expected values are to be measured, set by the information processing device 100. The graph 1100 in Fig. 11 depicts, by a solid line, a real part obtained when the quantum computer 201 calculates the Green's function so as to measure expected values represented by all of the quantum circuits 600, according to the conventional technique.

A graph 1110 depicted in Fig. 11 depicts, by a dotted line, an imaginary part obtained when the quantum computer 201 calculates the Green's function, based on the quantum circuits 600 whose expected values are to be measured, set by the information processing device 100. The graph 1110 of Fig. 11 depicts, by a solid line, an imaginary part obtained when the quantum computer 201 calculates the Green's function so as to measure expected values represented by all of the quantum circuits 600, according to the conventional technique.

Thus, in the case of the periodic boundary condition (PBC), the information processing device 100 may reduce the number of quantum circuits 600 whose expected values are to be measured, to 6/64 as compared with the conventional technique. At time t=0-30, the information processing device 100 may make the mean absolute error with respect to the Green's function obtained by the conventional technique, be $1.39 \times 10^{-14}$. Thus, the information processing device 100 may maintain the accuracy of the calculation of the Green's function, as compared with the conventional technique. It is to be noted here that occurrence of a difference in calculation result between the conventional technique and the information processing device 100 is attributable to the time evolution operator approximated by the Trotter decomposition not strictly meeting the Hamiltonian symmetry.

In this manner, the information processing device 100 may achieve reduction in number of the quantum circuits 600 whose expected values are to be measured when calculating the Green's function. Thus, the information processing device 100 may achieve reduction in processing burden and processing time imposed when calculating the Green's function. The information processing device 100 may avoid decreases in the accuracy of calculating the Green's function.

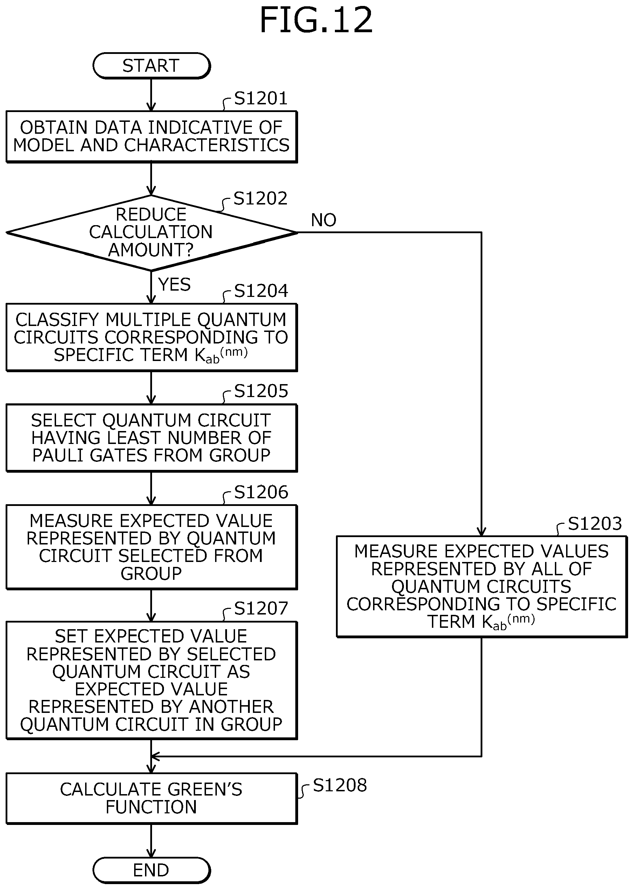

An example of an overall processing procedure that is executed by the information processing device 100 is described with reference to Fig. 12. The overall processing is implemented, for example, by the CPU 301, the storage region such as the memory 302 or the recording medium 305, and the network I/F 303.

Fig. 12 is a flowchart of an example of the overall processing procedure. In Fig. 12, the information processing device 100 obtains data indicating a model of a substance to be analyzed and characteristics that the substance has (step S1201).

The information processing device 100 then determines whether to reduce the calculation amount (step S1202). When affirmative (step S1202: YES), the information processing device 100 goes to processing at step S1204. On the other hand, when negative (step S1202: NO), the information processing device 100 goes to processing at step S1203.

At step S1203, the information processing device 100 measures expected values represented by all of quantum circuits corresponding to a specific term $K_{ab}^{(nm)}$ that forms the Green's function (step S1203). The information processing device 100 then goes to processing at step S1208.

At step S1204, the information processing device 100 classifies quantum circuits corresponding to the specific term $K_{ab}^{(nm)}$, based on the obtained data and thereby identifies multiple groups (step S1204). The information processing device 100 selects, for each of the groups, a quantum circuit 600 having the least number of Pauli gates in the group (step S1205).

The information processing device 100 controls, for each of the groups, the quantum computer 201 so as to measure the expected value represented by the quantum circuit selected from the group (step S1206). The information processing device 100 controls, for each of the groups, the quantum computer 201 so as to set the expected value represented by the selected quantum circuit as the expected value represented by another quantum circuit in the group (step S1207).

The information processing device 100 controls the quantum computer 201 to calculate the Green's function using expected values represented by all of the quantum circuits corresponding to the specific term $K_{ab}^{(nm)}$ that forms the Green's function (step S1208). The information processing device 100 then terminates the overall processing. The information processing device 100 may thus achieve reduction in processing burden and processing time imposed when calculating the Green's function. The information processing device 100 may calculate the Green's function with high accuracy.

Here, the information processing device 100 may interchange the order of processing at some steps in Fig. 12 and execute the processing. For example, the order of processing at step S1202 and at steps S1203 to S1205 may be interchanged. The information processing device 100 may omit processing at some steps in Fig. 12. For example, processing at step S1202 may be omitted.

As set forth hereinabove, according to the information processing device 100, depending on the characteristics of a substance, any quantum circuit may be selected from any group into which two or more quantum circuits are classified among multiple quantum circuits. According to the information processing device 100, the computing unit that calculates the Green's function may be controlled so that, by measuring the expected value represented by the selected quantum circuit, processing of measuring the expected value represented by another quantum circuit in the group is omitted. Thus, the information processing device 100 may achieve reduction in processing burden and processing time imposed when calculating the Green's function.

According to the information processing device 100, a quantum circuit having a relatively small number of Pauli gates may be selected from a group, based on the number of the Pauli gates that form each of two or more quantum circuits classified into the group. Thus, the information processing device 100 may easily reduce the processing burden and processing time imposed when calculating the Green's function.

According to the information processing device 100, for each of one or more groups into each of which two or more quantum circuits are classified, a quantum circuit may be selected from the group, depending on the characteristics a substance has. According to the information processing device 100, the computing unit may be controlled so that, by measuring the expected value represented by the selected quantum circuit, for each of the one or more groups, processing of measuring the expected value represented by another quantum circuit in the group is omitted. Thus, the information processing device 100 may reduce the processing burden and processing time imposed when calculating the Green's function.

According to the information processing device 100, a quantum circuit whose expected value is zero among multiple quantum circuits may be identified. According to the information processing device 100, the computing unit may be controlled to omit processing of measuring the expected value represented by the identified quantum circuit. Thus, the information processing device 100 may reduce the processing burden and processing time imposed when calculating the Green's function.

According to the information processing device 100, the computing unit may be controlled to employ the expected value represented by the measured quantum circuit in each group as the expected value represented by a quantum circuit different from the any quantum circuit in the each group. As a result, the information processing device 100 can make it possible for the computing unit to calculate the Green's function.

The information processing method described in the present embodiment may be implemented by executing a prepared program on a computer such as a personal computer and a workstation. The program is stored on a non-transitory, computer-readable recording medium such as a hard disk, a flexible disk, a compact-disc read-only memory (CD-ROM), a magneto optical disk (MO), and a digital versatile disc (DVD), read out from the computer-readable medium, and executed by the computer. The program may be distributed through a network such as the Internet.

According to one aspect, it becomes easy to calculate the Green's function using a quantum computer.

All examples and conditional language provided herein are intended for pedagogical purposes of aiding the reader in understanding the invention and the concepts contributed by the inventor to further the art, and are not to be construed as limitations to such specifically recited examples and conditions, nor does the organization of such examples in the specification relate to a showing of the superiority and inferiority of the invention. Although one or more embodiments of the present invention have been described in detail, it should be understood that the various changes, substitutions, and alterations could be made hereto without departing from the spirit and scope of the invention.

## Claims

1. A computer-readable recording medium storing therein an information processing program for causing a computer to execute a process, the process comprising:

    when calculating a Green's function by using a plurality of expected values represented respectively by a plurality of quantum circuits corresponding to a specific term that forms the Green's function, which is expressed in qubits and used when analyzing characteristics of a substance,
    selecting a first quantum circuit from a group into which two or more of the plurality of quantum circuits are classified when the plurality of quantum circuits is classified such that among the plurality of quantum circuits, quantum circuits corresponding to a same expected value are classified into a same group according to at least one of a plurality of characteristics of the substance, the plurality of characteristics including space-group symmetry, gauge symmetry, particle-hole symmetry, and time-reversal symmetry; and

controlling a computing device to calculate the Green's function so that, by measuring a first expected value represented by the selected first quantum circuit, the computing device omits measuring a second expected value represented by a second quantum circuit different from the selected first quantum circuit in the group.

2. The recording medium according to claim 1, wherein
the selecting includes selecting, based on numbers of Pauli gates that form each of the two or more quantum circuits classified into the group, the first quantum circuit having a relatively small number of the Pauli gates from the group.

3. The recording medium according to claim 2, wherein

the group is among a plurality groups into each of which two or more quantum circuits are classified when the plurality of quantum circuits is classified such that among the plurality of quantum circuits, quantum circuits corresponding to a same expected value are classified in a same group according to at least one of the plurality of characteristics of the substance, including the space-group symmetry, the gauge symmetry, the particle-hole symmetry, and the time-reversal symmetry,

the selecting includes selecting the first quantum circuit from each group of the plurality of groups, and

the controlling includes controlling the computing device for the each group so that, by measuring the first expected value represented by the selected first quantum circuit, the computing device omits measuring the second expected value represented by the second quantum circuit different from the selected first quantum circuit in the each group.

4. The recording medium according to any one of claims 1 to 3, the process further comprising

identifying a third quantum circuit whose expected value is zero among the plurality of quantum circuits, wherein the controlling includes controlling the computing device to omit measuring a third expected value represented by the identified third quantum circuit.

5. The recording medium according to any one of claims 1 to 3, wherein
the computing device uses the measured first expected value represented by the first quantum circuit in the group as the second expected value represented by the second quantum circuit different from the first quantum circuit.

6. An information processing method executed by a computer, the method comprising:

when calculating a Green's function by using a plurality of expected values represented respectively by a plurality of quantum circuits corresponding to a specific term that forms the Green's function, which is expressed in qubits and used when analyzing characteristics of a substance,

selecting a first quantum circuit from a group into which two or more of the plurality of quantum circuits are classified when the plurality of quantum circuits is classified such that among the plurality of quantum circuits, quantum circuits corresponding to a same expected value are classified into a same group according to at least one of a plurality of characteristics of the substance, the plurality of characteristics including space-group symmetry, gauge symmetry, particle-hole symmetry, and time-reversal symmetry; and

controlling a computing device to calculate the Green's function so that, by measuring a first expected value represented by the selected first quantum circuit, the computing device omits measuring a second expected value represented by a second quantum circuit different from the selected first quantum circuit in the group.

7. An information processing device, comprising:

a memory; and
a processor coupled to the memory, the processor configured to:

when calculating a Green's function by using a plurality of expected values represented respectively by a plurality of quantum circuits corresponding to a specific term that forms the Green's function, which is expressed in qubits and used when analyzing characteristics of a substance,

select a first quantum circuit from a group into which two or more of the plurality of quantum circuits are classified when the plurality of quantum circuits is classified such that among the plurality of quantum

**EP 4 435 676 A1**

circuits, quantum circuits corresponding to a same expected value are classified into a same group according to at least one of a plurality of characteristics of the substance, the plurality of characteristics including space-group symmetry, gauge symmetry, particle-hole symmetry, and time-reversal symmetry; and control a computing device to calculate the Green's function so that, by measuring a first expected value represented by the selected first quantum circuit, the computing device omits measuring a second expected value represented by a second quantum circuit different from the selected first quantum circuit in the group.

# FIG.1

GREEN'S FUNCTION

$$G_{ab}^{R}(t) = -2i\theta(t) \sum_{n,m} \lambda_a^{(n)} \lambda_b^{(m)*} Re \underbrace{\langle \psi_0 | U^\dagger P_a^{(n)} U P_b^{(m)} | \psi_0 \rangle}_{K_{ab}^{(nm)}}$$

QUANTUM CIRCUITS CORRESPONDING TO $K_{ab}^{(nm)}$

$|0\rangle$ —— $H$ —— $\circ$ —————— $\bullet$ —— $H$ —— 📈

$|\psi_0\rangle$ ———————— $P_b^{(m)}$ —— $U$ —— $P_a^{(n)}$ ————————

$U \approx e^{-iHt}$

CLASSIFY

GROUP

MEASURE    OMIT    OMIT

# FIG.2

# FIG.3

# FIG.4

401 **CPU**

402 **MEMORY**

201

400

403 **NETWORK I/F**

210 **NETWORK**

404 **RECORDING MEDIUM I/F**

405 **RECORDING MEDIUM**

406 **COMPUTING DEVICE I/F**

407 **QUANTUM COMPUTING DEVICE**

ROOM TEMPERATURE SIDE

ROOM TEMPERATURE SIDE

**MICROWAVE PULSE GENERATOR**

INPUT      OUTPUT

INPUT

**MICROWAVE PULSE DEMODULATOR**

OUTPUT

LOW NOISE AMPLIFIER

(10mK)

QUANTUM STATE TELEPORTATION

**QUBIT CHIP**

**QUBIT CHIP**

CRYOGENIC DILUTION REFRIGERATOR (HOUSING)

# FIG.5

# FIG.6

$$U \approx e^{-iHt}$$

# FIG.7

REVERSAL

700

710

701

# FIG.8

# FIG.9

900

DIAGONAL MIRROR

4-FOLD
ROTATION

VERTICAL MIRROR

# FIG.10

1000

N=16

2D HUBBARD MODEL

— CONVENTIONAL : $4N^2$

---- SYMMETRY−USING(OBC) : $N(N+2)/4$

−·− SYMMETRY-USING(PBC) : $(\sqrt{N}+2)(\sqrt{N}+4)/4$

NUMBER OF CIRCUITS

SITE NUMBER N

# FIG.11

TROTTER: N=2×2, U=10.0, μ=5.0, DEPTH=10   1100

1110

# FIG.12

START

↓

OBTAIN DATA INDICATIVE OF
MODEL AND CHARACTERISTICS ⟵ S1201

↓

REDUCE
CALCULATION
AMOUNT? ⟵ S1202 —— NO ——→

↓ YES

CLASSIFY MULTIPLE QUANTUM
CIRCUITS CORRESPONDING TO
SPECIFIC TERM $K_{ab}^{(nm)}$ ⟵ S1204

↓

SELECT QUANTUM CIRCUIT
HAVING LEAST NUMBER OF
PAULI GATES FROM GROUP ⟵ S1205

↓

MEASURE EXPECTED VALUE
REPRESENTED BY QUANTUM
CIRCUIT SELECTED FROM
GROUP ⟵ S1206

↓

SET EXPECTED VALUE
REPRESENTED BY SELECTED
QUANTUM CIRCUIT AS
EXPECTED VALUE
REPRESENTED BY ANOTHER
QUANTUM CIRCUIT IN GROUP ⟵ S1207

MEASURE EXPECTED VALUES
REPRESENTED BY ALL OF
QUANTUM CIRCUITS
CORRESPONDING TO SPECIFIC
TERM $K_{ab}^{(nm)}$ ⟵ S1203

↓

CALCULATE GREEN'S
FUNCTION ⟵ S1208

↓

END

Europäisches Patentamt

European Patent Office

Office européen des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TREVOR KEEN ET AL: "Hybrid quantum-classical approach for coupled-cluster Green's function theory", ARXIV.ORG, CORNELL UNIVERSITY LIBRARY, 201 OLIN LIBRARY CORNELL UNIVERSITY ITHACA, NY 14853, 7 February 2022 (2022-02-07), XP091144029, * abstract; figures 2, 3; table I * * page 1, column 1, line 17 - line 24 * * page 2, column 1, line 35 - line 41 * * page 3, column 2, line 1 - line 13 * * page 4, column 1, line 8 - page 6, column 2, line 25 * * page 17, line 3 - line 31 * | 1-7 | INV. G06N10/20 G06N10/60 |
| A | SUGURU ENDO ET AL: "Calculation of the Green's function on near-term quantum computers", ARXIV.ORG, CORNELL UNIVERSITY LIBRARY, 201 OLIN LIBRARY CORNELL UNIVERSITY ITHACA, NY 14853, 5 August 2020 (2020-08-05), XP081733628, * abstract * * page 4, column 2, line 1 - page 5, column 1, line 5 * | 1-7 | |
| | | | TECHNICAL FIELDS SEARCHED (IPC) G06N |
| A | DIOGO CRUZ ET AL: "Super-resolution of Green's functions on noisy quantum computers", ARXIV.ORG, CORNELL UNIVERSITY LIBRARY, 201 OLIN LIBRARY CORNELL UNIVERSITY ITHACA, NY 14853, 10 October 2022 (2022-10-10), XP091339179, * abstract; figures 1, 2 * * page 1, column 1, line 1 - page 4, column 2, line 4 * | 1-7 | |

-/--

The present search report has been drawn up for all claims

| Place of search | Date of completion of the search | Examiner |
|---|---|---|
| The Hague | 9 July 2024 | Rousset, Antoine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 16 1994

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FRANCOIS JAMET ET AL: "Krylov variational quantum algorithm for first principles materials simulations", ARXIV.ORG, CORNELL UNIVERSITY LIBRARY, 201 OLIN LIBRARY CORNELL UNIVERSITY ITHACA, NY 14853, 26 August 2021 (2021-08-26), XP091026042, * abstract; figure 1 * * page 1, column 1, line 1 - page 5, column 1, line 12 * ----- | 1-7 | |

TECHNICAL FIELDS
SEARCHED      (IPC)

The present search report has been drawn up for all claims

| Place of search | Date of completion of the search | Examiner |
|---|---|---|
| The Hague | 9 July 2024 | Rousset, Antoine |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021068281 A
- JP 2022007590 A